# EUROPÄISCHE PATENTSCHRIFT

(11) **EP 0 925 081 B1**
(45) Veröffentlichungstag und Bekanntmachung des Hinweises auf die Patenterteilung: **03.12.2003**
(21) Anmeldenummer: 98921408.5
(22) Anmeldetag: 31.03.1998
(51) Int. Cl.: A61M 1/10

(54) **INTRAKARDIALE PUMPVORRICHTUNG**
INTRACARDIAC PUMP DEVICE
DISPOSITIF A POMPE INTRACARDIAQUE

(30) Priorität: 02.04.1997 US 832040
(43) Veröffentlichungstag der Anmeldung: 30.06.1999
(73) Patentinhaber: Impella Cardiotechnik Aktiengesellschaft, 52074 Aachen (DE)
(72) Erfinder: SIESS, Thorsten, D-52146 Würselen (DE)
(74) Vertreter: Selting, Günther, Dipl.-Ing.
(86) Internationale Anmeldenummer: EP9801868
(87) Internationale Veröffentlichungsnummer: WO98043689

(56) Entgegenhaltungen:
- WO-A-94/09835
- DE-A- 2 113 986
- US-A- 4 662 355
- YAMAZAKI K ET AL: "DEVELOPMENT OF A MINIATURE INTRAVENTRICULAR AXIAL FLOW BLOOD PUMP" ASAIO JOURNAL, Bd. 39, Nr. 3, 1. Juli 1993, Seiten M224-M230, XP000412587

## Beschreibung

Die Erfindung betrifft eine intrakardiale Pumpvorrichtung mit zwei Pumpen, die in das Herz eingeführt werden können, um die natürliche Pumpfunktion des Herzens zu unterstützen oder durch kontinuierlichen Pumpbetrieb zu ersetzen.

Eine Pumpvorrichtung für die Herzunterstützung ist beschrieben in WO94/09835 (Jarvik). Diese Pumpvorrichtung weist zwei voneinandner unabhängige Pumpen auf, die jeweils aus einem Pumpenteil und einem damit starr verbundenen Antriebsteil bestehen. Der Pumpenteil der einen Pumpe wird durch eine apekale Operationsöffnung hindurch derart in den linken Ventrikel eingeführt, daß er aus dem linken Ventrikel heraus in die Aorta fördert. Der andere Pumpenteil wird durch eine weitere Operationsöffnung hindurch in den rechten Ventrikel so eingeführt, daß er aus dem rechten Ventrikel in die Pulmonalarterie hineinfördert. Das System enthält weiterhin einen Kontroll- und Anzeigemodul, der klein genug ist, um sterilisiert und im sterilen Operationsbereich benutzt zu werden. Dieser kann einen Mikroprozessor mit Kontroll- und Überwachungsalgorithmen enthalten, um den Volumenstrom und Druck zu regulieren, oder um den Volumenstrom und Druck, die durch Sensoren gemessen oder durch Vergleich der Messungen von Geschwindigkeit und Energieaufnahme berechnet wurden, einer Datenbasis zuzuführen. Die als Kanülenpumpen bezeichneten Pumpen können mit eingebauten Drucksensoren oder Volumenstrommessvorrichtungen ausgestattet sein, um örtliche Messungen dieser Parameter im Rahmen des Patientenmanagement durchzuführen.

US-A-4,662,355 beschreibt eine Pumpenregelvorrichtung für zwei extrakorporale Pumpen zur Herzunterstützung. Diese Pumpen sind peristaltische Schlauchpumpen, die von einer gemeinsamen Regeleinrichtung in gegenseitiger Abhängigkeit derart gesteuert werden, dass sie physiologisch identische Volumenströme fördern. Durch ein Potentiometer kann die Proportion der beiden Volumenströme verändert werden. Volumenstrom-Messeinrichtungen sind hierbei nicht vorhanden.

In einem Aufsatz von YAMAZAKI K ET AL: "DEVELOPMENT OF A MINIATURE INTRAVENTRICULAR AXIAL FLOW BLOOD PUMP" ASAIO JOURNAL, Bd. 39, Nr. 3, 1. Juli 1993, Seiten M224-M230, XP000412587, sind Messungen an einer intraventrikularen Axialstrom-Blutpumpe beschrieben. Dabei werden die Drücke am Pumpeneinlass und am Pumpenauslass gemessen. Ein Volumenstrommesser misst zusätzlich den Volumenstrom am Pumpenauslass. Dabei ergibt sich eine lineare Charakteristik des Differenzialdrucks in Abhängigkeit vom Volumenstrom bei unterschiedlichen Pumpendrehzahlen, wenn die Pumpe kontinuierlich läuft und keine Unterstützung durch das schlagende Herz erfolgt. Aus der Veröffentlichung geht auch hervor, dass bei Anwendung der Pumpe beim schlagenden Herzen die Linearität zwischen Differenzialdruck und Volumenstrom nicht mehr existiert und durch eine ausgeprägte Hysteresekurve ersetzt wird.

Der vorliegenden Erfindung liegt die Aufgabe zugrunde, eine intrakardiale Pumpvorrichtung mit mindestens zwei Pumpen zu schaffen, bei der eine wirksame Herzunterstützung unter geeigneter Bemessung der Betriebsbedingungen beider Pumpen erzielbar ist.

Die erfindungsgemäße intrakardiale Pumpvorrichtung gemäß Anspruch 1 enthält zwei im Herzen zu platzierende Pumpen, von denen jede eine Antriebseinheit und eine damit starr verbundene Pumpeneinheit aufweist. Beide Pumpen sind von einer gemeinsamen Steuereinheit in gegenseitiger Abhängigkeit betrieben. Die beiden Pumpen werden nicht unabhängig voneinander betrieben, sondern in der Weise, dass bei beiden Pumpen die für den Pumpenbetrieb wichtigen Parameter aufeinander abgestimmt sind. Dadurch wird ein interferierender Betrieb beider Pumpen vermieden und es wird auch verhindert, dass unnatürliche Druckoder Saugverhältnisse im Herzen und den peripheren Organen auftreten können.

Intrakardial im Sinne der vorliegenden Erfindung umfasst die Herzkammern (Ventrikel), die Vorhöfe und die angrenzenden Gefäßstümpfe. Ein wesentlicher Parameter einer intrakardialen Blutpumpe ist der pro Zeiteinheit geförderte Volumenstrom. Die Steuerungen beider Pumpen sind so aufeinander abgestimmt, daß die Volumenströme in einem vorbestimmten Verhältnis zueinander stehen. Wenn die eine Pumpe als Rechtsherzpumpe und die andere als Linksherz-Pumpe eingesetzt wird, fördern beide Pumpen in Fluidsysteme, die in Strömungsrichtung in Reihe hintereinander angeordnet sind, wobei zuerst das mit dem rechten Ventrikel in direkter Fluidkommunikation stehende System zur Versorgung der Lunge durchströmt wird. Nach Oxigenierung des Blutes in der Lunge kehrt das Blut zum Herzen zurück, und zwar in den linken Ventrikel. Vom linken Ventrikel wird das Blut in die Aorta gepumpt. Im peripheren Gefäßsystem reduziert sich die Blutmenge jedoch durch Verzweigungen und "Leckverluste" um etwa 10 %. Der Volumenstrom der Rechtsherzpumpe ist um einen bestimmten Prozentsatz, vorzugsweise etwa 10 %, kleiner als derjenige der Linksherz-Pumpe. Dieser festgelegte Prozentsatz bleibt bei einer Änderung des Volumenstromes erhalten, d.h. die Volumenströme beider Pumpen ändern sich in gleichen prozentualen Ausmaßen.

Die gemeinsame Steuereinheit zur Steuerung beider Pumpen kann auch aus unterschiedlichen Steuereinrichtungen bestehen, die miteinander kommunizieren, so daß eine Änderung des Volumenstroms einer Pumpe automatisch zu einer entsprechenden Änderung des Volumenstromes der anderen Pumpe führt.

Vorzugsweise sind beide Pumpen in Master-Slave-Steuerung betrieben, wobei normalerweise die Linksherz-Pumpe die Funktion des Masters hat. Die Linksherz-Pumpe ist diejenige, die die größte Fördermenge gegen den höchsten Gegendruck zu bewältigen hat und die daher am höchsten beansprucht wird.

Beide Pumpen können einander gleich ausgebildet sein und somit gleiche Bauformen und Leistungscharakteristiken haben.

Die Pumpen sind vorzugsweise als intravasale Pumpen ausgebildet, wie sie in der (nicht vorveröffentlichten) WO97/37696 beschrieben sind. Eine solche intravasale Blutpumpe ist mit einem Katheter verbunden. Sie hat so geringe Abmessungen, daß sie durch ein Blutgefäß hindurch an den Einsatzort geschoben werden kann, oder auch im Blutgefäß betrieben werden kann. Bei einer solchen intravasalen Blutpumpe haben der Pumpenteil und der Antriebsteil im wesentlichen gleichen Durchmesser, der nicht größer ist als etwa 5 - 7 mm, da die Gefäßweite in den Körperrandbereichen maximal etwas mehr als 7 mm beträgt. Die rigide Länge einer solchen Pumpe darf nicht mehr als etwa 35 mm betragen, damit die Pumpe durch die Krümmungen von Blutgefäßen hindurchgeht. Allerdings kann die Pumpe zusätzlich mit einem flexiblen Schlauch verlängert sein, der die effektive Länge der Pumpe vergrößert.

Andererseits besteht die Möglichkeit, die Pumpe operativ über das herznahe Gefäßsystem in das Herz einzuführen. In jedem Fall sind die Pumpen so klein, daß sie in das Herz, einschließlich der Vorhöfe und der angrenzenden Gefäßstümpfe, hineinpassen und im Herzen betrieben werden können, ohne daß Teile der Pumpe aus dem Herzen herausragen. Aus dem Herzen oder den Blutgefäßen herausgeführt sind allenfalls die Katheter, die mit den Pumpen verbunden sind. Diese Katheter enthalten nicht nur die Leitungen zum Zuführen elektrischer Energie zu den Pumpen, sondern auch die Signalleitungen, die von den an den Pumpen vorgesehenen Sensoren zu der extrakorporalen Steuereinheit führen.

Im folgenden werden Ausführungsbeispiele der Erfindung unter Bezugnahme auf die Zeichnungen näher erläutert.

Es zeigen:
- Fig. 1: einen schematischen Längsschnitt durch eine Ausführungsform einer intrakardialen Blutpumpe,
- Fig. 2: ein Ausführungsbeispiel der intrakardialen Anwendung zweier intravasaler Pumpen,
- Fig. 3: ein Ausführungsbeispiel der Anwendung zweier durch die Blutgefäßwandungen hindurch operativ in das Herz eingeführter Pumpen,
- Fig. 4: ein schematisches Diagramm zur Erläuterung des Betriebes der beiden Pumpen,
- Fig. 5: ein Diagramm zur Verdeutlichung der Abhängigkeit des Volumenstromes von der Druckdifferenz zwischen der Saugseite und der Druckseite einer Pumpe,
- Fig. 6: ein Blockschaltbild der Steuerung der beiden Pumpen und
- Fig. 7: ein Diagramm des zeitlichen Verlaufs verschiedener Drücke im Herzen.

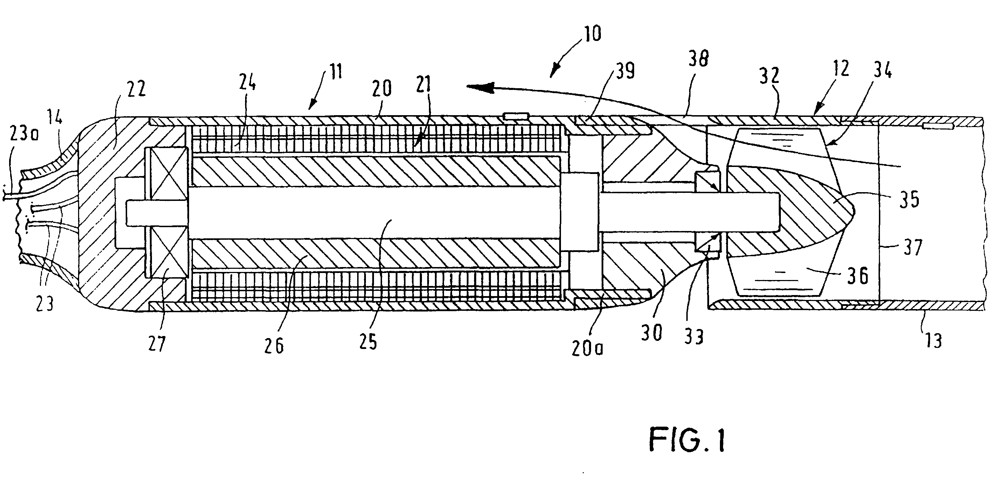

In Fig. 1 ist eine intravasale Blutpumpe 10 dargestellt, also eine Blutpumpe, die durch das Blutgefäßsystem eines Patienten geschoben werden kann, um bis in das Herz hinein vorzudringen. Der Außendurchmesser einer solchen Blutpumpe ist an keiner Stelle größer als 7 mm.

Die Pumpe 10 weist einen Antriebsteil 11 und einen damit starr verbundenen Pumpenteil 12 auf. Der Antriebsteil 11 weist ein langgestrecktes zylindrisches Gehäuse 20 auf, in dem ein Elektromotor 21 untergebracht ist. An dem rückwärtigen Ende ist das Gehäuse 20 mit einer Stirnwand 22 verschlossen, an die sich ein flexibler Katheter 14 abdichtend anschließt. Durch den Katheter 14 verlaufen die elektrischen Kabel 23 zur Stromversorgung und zur Steuerung des Elektromotors 21, und außerdem weitere Kabel 23a, die mit Sensoren der Pumpe 10 verbunden sind.

Der Stator 24 des Motors weist in üblicher Weise zahlreiche umfangsmäßig verteilt angeordnete Wicklungen sowie einen magnetischen Rückschluß in Längsrichtung auf. Er ist mit dem Motorgehäuse 20 fest verbunden. Der Stator 24 umgibt den mit der Motorwelle 25 verbundenen Rotor 26, der aus in Wirkrichtung magnetisierten Permanentmagneten besteht. Die Motorwelle ist am rückwärtigen Ende mit einem Lager 27 im Motorgehäuse bzw. in der Stirnwand 22 gelagert. Die Motorwelle erstreckt sich durch die gesamte Länge des Motorgehäuses 20 und ragt nach vorne aus diesem heraus.

Den vorderen Abschluß des Motorgehäuses bildet ein rohrförmiges stationäres Nabenteil 30, das mit seinem rückwärtigen Ende in einem Ansatz 20a von verringertem Durchmesser des Gehäuses 20 sitzt. Der Außendurchmesser des Nabenteils verjüngt sich zum vorderen Ende hin, wo sich ein Lager 33 zur Lagerung der Motorwelle 25 befindet. Dieses Lager ist zugleich als Wellendichtung ausgebildet.

Die Motorwelle 25 steht aus dem Nabenteil 30 nach vorne vor und trägt dort ein Flügelrad 34 mit einer auf dem Wellenende sitzenden Nabe 35 und davon abstehenden Flügeln 36 oder Pumpenschaufeln, die in bezug auf die Achse des Flügelrades 34 schräggestellt sind. Das Flügelrad 34 ist in einem zylindrischen Pumpengehäuse 32 enthalten, das durch drei umfangsmäßig verteilte Stege 38 mit dem Motorgehäuse 20 verbunden ist. Das Motorgehäuse 20 und das Pumpengehäuse 32 sind durch einen Ring 39 starr miteinander verbunden und haben gleiche Außendurchmesser. Der Durchmesser der Pumpe 10 ist an keiner Stelle größer ist als dieser Außendurchmesser.

Bei einer Rotation des Flügelrades 34 wird Blut durch die Ansaugöffnung 37 des Pumpengehäuses 32 angesaugt und in axialer Richtung im Pumpengehäuse 32 nach hinten getrieben. Durch den ringförmigen Spalt zwischen dem Pumpengehäuse 32 und dem Motorgehäuse 20 strömt das Blut am Nabenteil 30 entlang nach außen, um weiter an dem Motorgehäuse 20 entlangzuströmen. Hierdurch wird der Abtransport der im Antrieb erzeugten Wärme sichergestellt, ohne daß es zur Blutschädigung durch zu hohe Oberflächentemperaturen (über 41 °C) auf dem Motorgehäuse 20 kommt.

Es ist auch möglich, den Pumpenteil 12 für die umgekehrte Förderrichtung auszulegen, wobei das Blut an dem Motorgehäuse entlang angesaugt wird und aus der stirnseitigen Öffnung 37 axial austritt.

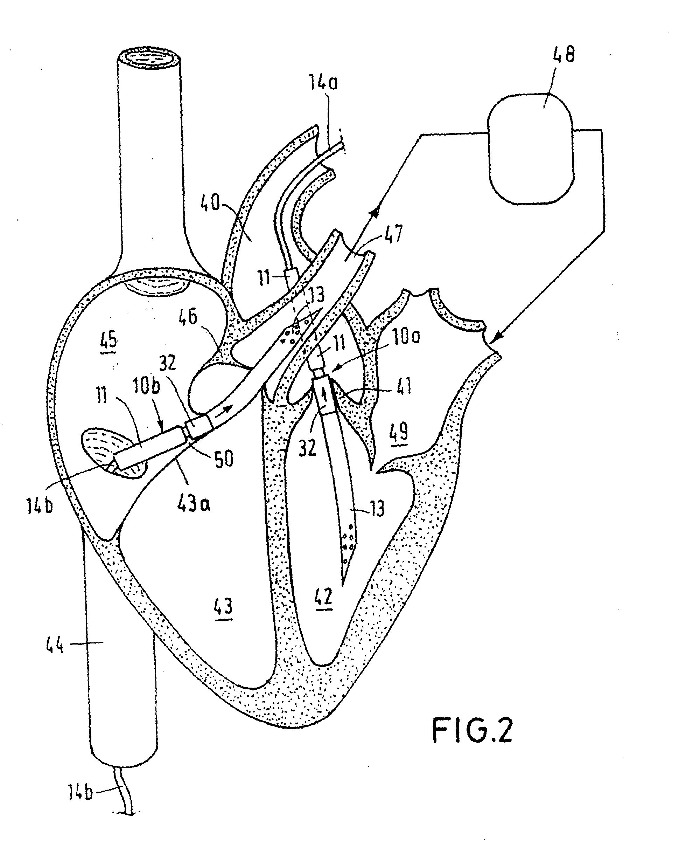

Fig. 2 zeigt ein Ausführungsbeispiel der Verwendung zweier generell einander gleicher Pumpen 10a,10b, die gemäß Fig. 1 ausgebildet sind, in einem Herzen, zur Herzunterstützung oder als Ersatz für die Herzpumpfunktion bei stillgesetztem Herzen. Beide Pumpen 10a,10b sind jeweils mit einem Katheter 14a,14b verbunden. Sie sind percutan verlegt worden, wobei der Katheter 14a der Linksherz-Pumpe 10a durch die Aorta 40 verläuft und ein die Pumpe 10a verlängernder Schlauch 13 durch die Aortenklappe 41 in den linken Ventrikel 42 hinein vorgeschoben ist. Dabei ist der Pumpenteil 12 mit dem mit dem Pumpengehäuse 32 verbundenen flexiblen Schlauch 13 verlängert, der an seinem Ende und/oder in seiner Seitenwand Öffnungen für den Blutzutritt zur Pumpe 10a aufweist. Die Pumpe 10a saugt durch den Schlauch 13 an und fördert das Blut in die Aorta 40, während die Aortenklappe 41 sich von außen gegen das Pumpengehäuse 32 bzw. den Schlauch 13 legt. Im linken Ventrikel 42 wird somit die Pumpe 10a als Linksherz-Pumpe mit axialer Ansaugung betrieben.

Die andere Pumpe 10b wird als Rechtsherzpumpe in Fluidkommunikation mit dem rechten Ventrikel 43 betrieben. Der Katheter 14b führt durch die obere oder die untere Hohlvene 44 hindurch in das rechte Atrium 45. Der Schlauch 13 der Pumpe 10b ragt durch die Tricuspidalklappe 43a und die Pulmonalklappe 46 in die Pulmonalarterie 47, von der das Blut zur Oxigenierung zur Lunge 48 fließt. Das oxigenierte Blut fließt dann in den linken Vorhof 49 und von dort in den linken Ventrikel 42.

Die Pumpe 10b saugt durch den radialen Einlaß 50 an und fördert das Blut durch den Schlauch 13 axial in die Pulmonalarterie 47 hinein. Die Pumpe 10b wird also invers zu der Pumpe 10a betrieben.

Beide Pumpen 10a,10b werden in das Herz eingeführt, ohne daß einer der Ventrikel operativ geöffnet werden müßte.

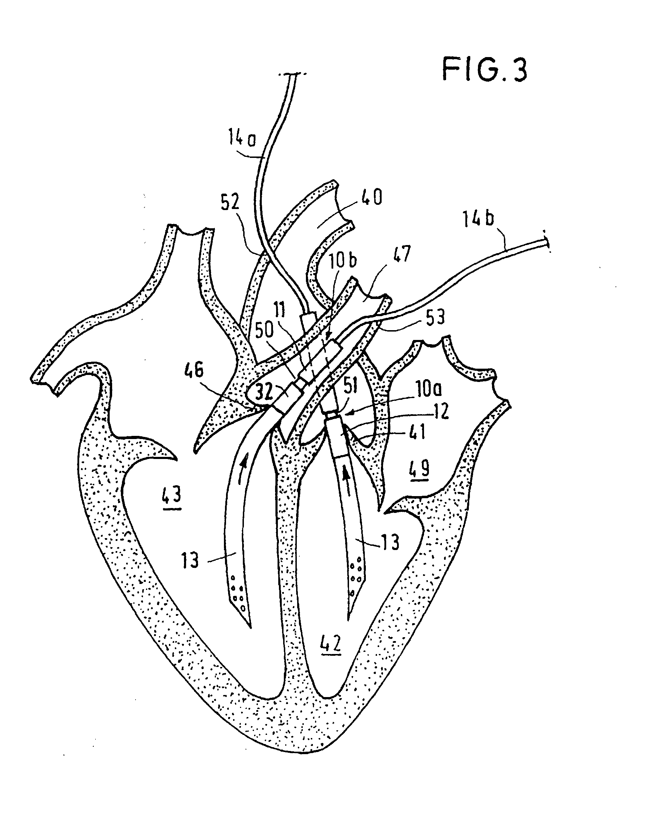

Fig. 3 zeigt eine andere Möglichkeit der Plazierung zweier Pumpen im Herzen, mit der Fähigkeit einer Herzunterstützung oder auch eines Ersatzes der gesamten Pumpfunktion des Herzens. Beide Pumpen 10a,10b sind im Gegensatz zu Fig. 2 operativ durch Incisionen des Gefäßsystems in das Herz implantiert worden, wobei der Katheter 14a der Linksherz-Pumpe 10a durch die Aortenwand 52 hindurchgeht, während der Katheter 14b der Rechtsherz-Pumpe 10b bei 53 durch die Wand der Pulmonalarterie 47 hindurchgeht. Der Schlauch 13 der Linksherz-Pumpe 10a ist in dem linken Ventrikel 42 angeordnet und distal von der Auslaßöffnung 51 von der Aortenklappe 41 umschlossen. Der Schlauch 13 der Rechtsherz-Pumpe 10b ist im rechten Ventrikel 43 angeordnet und von der Pulmonalklappe 46 umschlossen. Beide Pumpen 10a,10b werden so betrieben, daß sie durch ihre Schläuche 13 ansaugen und durch die Auslaßöffnungen 50 und 51 ausstoßen.

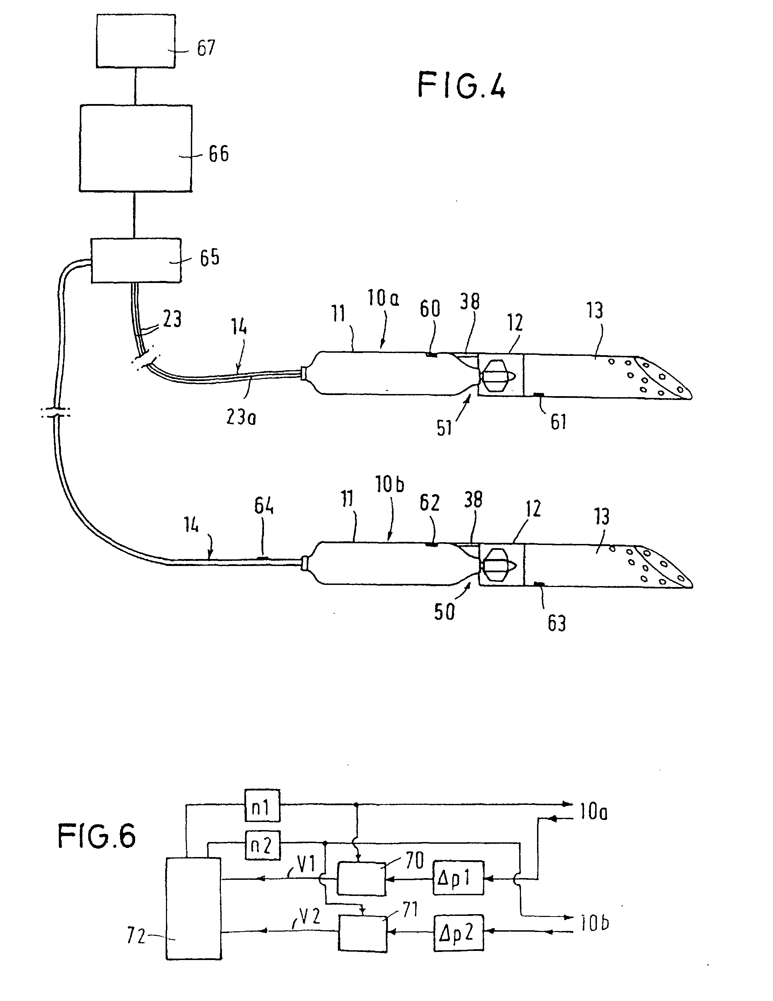

Fig. 4 zeigt eine schematische Darstellung der beiden Pumpen 10a,10b der vorherigen Ausführungsbeispiele mit verschiedenen Sensoren. Im einzelnen ist an der Außenfläche der Antriebseinheit 11 ein erster Drucksensor 60 vorgesehen, der nahe der radialen Öffnung 51 angeordnet ist, während ein zweiter Drucksensor 61 in der Nähe des Einlasses des Pumpengehäuses angeordnet ist. Die Leitungen 23a der Sensoren sind in die Komponenten der Pumpe integriert und verlaufen gemeinsam mit den Versorgungsleitungen 23 durch den Katheter 14. Der Drucksensor 60 hat seine Sensorfläche an der Außenseite des Motorgehäuses. Dagegen befindet sich die Sensorfläche des Drucksensors 61 an der Innenseite des Schlauchs 13. Ferner kann an dem Antriebsteil ein Temperatursensor angebracht sein, der die Motortemperatur überwacht.

Bei der Pumpe 10b ist ebenfalls ein erster Drucksensor 62 an der Außenseite des Motorgehäuses und ein weiterer Drucksensor 63 an der Innenseite des Schlauchs 13 angeordnet. Die Leitungen dieser Sensoren führen ebenfalls durch den Katheter 14 hindurch. Am Katheter 14 ist ein Sauerstoffsensor 64 angebracht, der Informationen über die Sauerstoffsättigung des Blutes liefert.

Die Versorgungsleitungen 23 und die Leitungen 23a stehen mit einem extrakorporalen Interface 65 in Verbindung. Dieses liefert die Signale der Sensoren an eine Steuereinheit 66, die diese Signale auswertet und in Abhängigkeit davon die Pumpen 10a,10b steuert. Eine Tastatur- und Anzeigevorrichtung 67 ist an die Steuereinheit 66 angeschlossen, um Informationen einzugeben und anzeigen zu können.

Mit den von den Sensoren gelieferten Informationen ist es möglich, die Position einer Pumpe in bezug auf ein externes Umschließungsteil, z.B. eine Herzklappe, festzustellen. Wenn sich Pumpeneinlaß und Pumpenauslaß auf unterschiedlichen Seiten des Umschließungsteils befinden, tritt an den Drucksensoren wegen der unterschiedlichen Druckzustände eine Druckdifferenz auf. Wenn das Herz schlägt, variiert diese Druckdifferenz auch zeitlich. Andererseits sind gleiche Druckmeßwerte ein Anzeichen für eine falsche Pumpenplazierung, da dann beide Drucksensoren denselben Druck messen. Die Auswertung der durch die beiden Drucksensoren gelieferten Daten unter Berücksichtigung des Motorstromes liefert wichtige Informationen über die Anordnung und Operation einer Pumpe. Durch Vergleichen der Druckdifferenz mit dem aktuellen Motorstrom ist es auch möglich, Blockadezustände oder Kavitation festzustellen.

Die Information über den Einlaß- und Auslaßdruck der Pumpe zusammen mit dem Energieverbrauch des Elektromotors liefert wichtige Aussagen über die Funktion der Pumpvorrichtung. Sie liefert auch eine Realzeitangabe über den Volumenstrom und ermöglicht eine Plazierung der Pumpe ohne Röntgenkontrolle oder Ultraschallkontrolle. Ferner kann eine Realzeitüberwachung behinderter Einlaßströmung erfolgen, wie sie z. B. durch Kollabieren des Ventrikels, Thrombenbildung, Okklusion des Schlauchs oder Ansaugen von Herzgewebe hervorgerufen wird. Die Sensoren bieten auch die Möglichkeit, Lagerverschleiß oder Versagen des Motors zu überwachen oder solche Ereignisse vorauszusagen. Ferner kann der Betrieb der Pumpe mit akzeptablen Gesamthämolyseraten über den erforderlichen Benutzungszeitraum und mit dem erforderlichen Volumenstrom von 3,5 bis 5 l/min. aufrechterhalten werden. Die Leistungstrends verschiedener Parameter können über mehrere Betriebsstunden hinweg angezeigt und analysiert werden, wobei Alarmzustände, die ein unverzügliches Eingreifen erfordern, erkannt werden, ohne daß eine permanente Überwachung durch Personen erforderlich ist. Ferner kann das Herz eines Patienten überwacht werden, ohne daß die Pumpe entfernt wird. Mit der Plazierung zweier instrumentierter Pumpen ist es möglich, der Steuereinheit die von einer Pumpe gelieferten örtlichen Informationen zu liefern, um den Betrieb der anderen Pumpe zu steuern und dabei die Leistungsfähigkeit des Systems als Ganzes zu optimieren.

Die Steuereinheit 66 steuert die beiden Pumpen 10a,10b in der Weise, daß jede der beiden Pumpen einen bestimmten volumenstrom (Blutvolumen pro Zeiteinheit) liefert. Dabei pumpt die Rechtsherz-Pumpe 10b einen bestimmten prozentualen Anteil des Volumenstroms der Linksherz-Pumpe 10a, beispielsweise 90 %. Der Volumenstrom der Rechtsherz-Pumpe ist immer kleiner als derjenige der Linksherz-Pumpe. Primär wird die Pumpenleistung der Linksherz-Pumpe 10a derart geregelt, daß ein bestimmter gewünschter Volumenstrom aufrechterhalten wird. In Abhängigkeit hiervon wird anschließend die Pumpenleistung der Rechtsherz-Pumpe 10b bestimmt. Hierbei handelt es sich um einen Master-Slave-Betrieb, bei dem in der Regel die Linksherz-Pumpe 10a der Master und die Rechtsherz-Pumpe 10b der Slave ist.

Die Pumpen werden von Synchronmotoren angetrieben, wobei die Steuereinheit 66 die erforderliche Antriebsfrequenz oder Drehzahl n liefert. Der Volumenstrom jeder Pumpe hängt von der Drehzahl n ab.

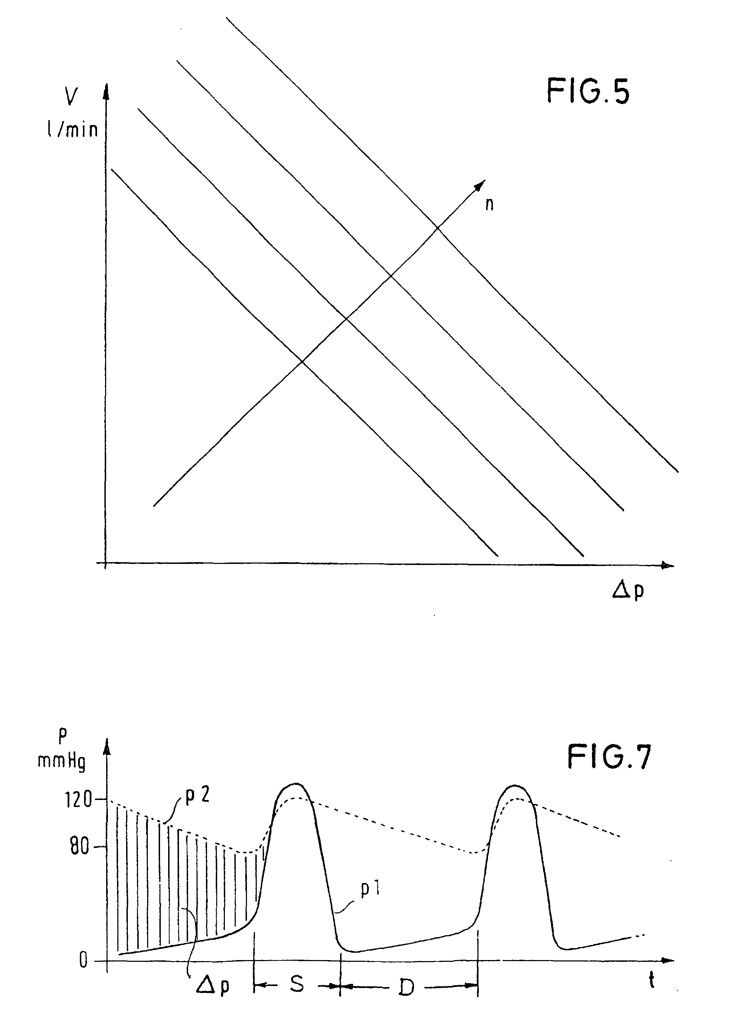

Fig. 5 zeigt den Volumenstrom V einer Pumpe in Abhängigkeit von der Druckdifferenz ΔP zwischen der Saugseite und der Druckseite der Pumpe jeweils für unterschiedliche Drehzahlen n. Jede der parallelen Geraden bezieht sich auf eine bestimmte Drehzahl n. Man erkennt, daß aus der Druckdifferenz ΔP der Volumenstrom V ermittelt werden kann, wenn die Drehzahl n bekannt ist. Der Motor 21 ist ein elektronisch kommutierter Synchronmotor. Da die Drehzahl von der Steuereinheit vorgegeben wird, ist die Drehzahl bekannt. Die Druckdifferenz ΔP wird mit den Sensoren 60 und 61 bzw. 62 und 63 ermittelt. Außerdem werden natürlich auch die Absolutwerte der Drücke gemessen und ausgewertet.

Wenn eine Pumpe durch die radiale Ansaugöffnung 50 bzw. 51 ansaugt und in den Schlauch 13 hinein fördert, ist der Druck an dem schlauchseitigen Drucksensor 61 bzw. 63 größer als an dem saugseitigen Drucksensor 60 bzw. 62. Fördert die Pumpe dagegen in umgekehrter Richtung, d.h. saugt sie durch den Schlauch 13 an, so ist der Druck an dem Drucksensor 60 bzw. 62 höher als derjenige an dem Drucksensor 61 bzw. 63.

Das in Fig. 6 dargestellte Diagramm verdeutlicht die Ermittlung der Volumenströme V1 und V2. Es wird die Druckdifferenz ΔP1 an der ersten Pumpe und die Druckdifferenz ΔP2 an der zweiten Pumpe gemessen. Einer ersten Auswerteeinrichtung 70 wird die Drehzahl n1 der Pumpe 10a und außerdem die Druckdifferenz ΔP1 zugeführt. Einer zweiten Auswerteeinrichtung 71 wird die Drehzahl n2 der zweiten Pumpe 10b und die Druckdifferenz ΔP2 an dieser zweiten Pumpe zugeführt. Die Auswerteeinrichtungen 70,71 liefern daraufhin anhand des Diagramms von Fig. 5 die Volumenströme V1 und V2 der ersten und der zweiten Pumpe an die Steuereinrichtung 72, die daraufhin die Drehzahlen n1 und n2 derart festsetzt, daß die Volumenströme V1 und V2 vorbestimmte Werte annehmen. Bei dem beschriebenen Beispiel werden die Volumenströme für die beiden Pumpen in gegenseitiger Abhängigkeit so festgelegt, daß der Volumenstrom V2 90 % des Volumenstroms von V1 beträgt. Die Einhaltung bzw. Regelung der Volumenströme erfolgt für beide Pumpen jeweils unabhängig.

Es kann vorkommen, daß eine der Pumpen durch Ansaugen am Herzgewebe oder am Klappenapparat ganz oder teilweise okkludiert wird. In diesem Fall liefern die Drucksensoren abnormale Werte. Dann wird die betreffende Pumpe für eine Zeit in der Drehzahl reduziert, so daß sich das Herzgewebe lösen kann, und anschließend wieder auf die gewünschte Drehzahl erhöht. Wenn der gemessene Absolutdruck zu groß wird, führt die Steuereinheit 66 eine Begrenzung - und ggf. eine Herabsetzung - des Volumenstromes durch, um nachfolgende Organe (Lunge) nicht zu schädigen.

Durch die Druckmessung erfolgt auch eine Wächterfunktion. Der Druck im rechten Ventrikel bzw. in der Pulmonalarterie darf einen bestimmten Wert nicht überschreiten und der Druck im linken Ventrikel bzw. in der Aorta darf einen bestimmten Druck nicht unterschreiten. Werden entsprechende Druckabweichungen festgestellt, wird Alarm gegeben oder nachgeregelt.

In Fig. 7 ist der Druckverlauf p1 im linken Ventrikel des schlagenden Herzens mit den Systolen S und den Diastolen D dargestellt. Man erkennt einen stark pulsierenden Druck, der zwischen den Systolen S stark abfällt. Außerdem ist der Druckverlauf p2 in der Aorta dargestellt. Der Aortendruck pulsiert zwar auch, jedoch in einem viel engeren Druckbereich. Die Druckdifferenz ΔP bestimmt sich zu p2 - p1. Diese Druckdifferenz kann mit den an den Pumpen vorgesehenen Drucksensoren bestimmt werden.

Die bisher beschriebenen Sensoren 61-63 sind Absolutdrucksensoren. Es besteht auch die Möglichkeit, an dem Pumpengehäuse 32 einen Differenzdrucksensor in Form einer Membran vorzusehen, die an ihrer Innenseite mit einem anderen Druck beaufschlagt wird als an ihrer Außenseite. Auf diese Art kann die Druckdifferenz jeweils mit einem einzigen Sensor ermittelt werden.

Die Messung der Drücke und Druckdifferenzen ist insbesondere auch für das Einführen der Pumpe in die richtige Position im Herzen wichtig. Das Einführen kann in der Weise geschehen, daß die Pumpe stillgesetzt ist oder mit einer geringen Drehzahl läuft, während das Herz schlägt. Wenn der eine Drucksensor den stark pulsierenden Druckverlauf p1 und der andere den weniger stark pulsierenden Druckverlauf p2 feststellt, ist die Pumpe korrekt plaziert.

Zur Plazierung ist eine Druckmessung jedoch nicht erforderlich. Vielmehr kann die Plazierung auch anhand des Stromverlaufs der Pumpe überwacht werden. Solange sich Einlaß und Auslaß einer Pumpe in demselben Raum befinden, sind beide demselben Druck ausgesetzt. Wird die Pumpe mit einer bestimmten Drehzahl angetrieben, so ist der zeitliche Verlauf des Pumpenstromes konstant. Befinden sich dagegen Auslaß und Einlaß der Pumpe in unterschiedlichen Räumen mit variierenden zeitlichen Druckverläufen, ergibt sich ein nicht glatter pulsierender Pumpenstrom. Anhand des Pumpenstroms kann somit festgestellt werden, ob die Herzklappe das Pumpengehäuse bzw. den Schlauch ordnungsgemäß umschließt, so daß der Einlaß der Pumpe sich in dem Ventrikel bzw. Vorhof und der Auslaß in der Aorta bzw. der Pulmonalarterie befindet.

## Patentansprüche

1. Intrakardiale Pumpvorrichtung mit zwei im Herzen zu platzierenden Pumpen (10a,10b), von denen jede eine Antriebseinheit (11) und eine starr damit verbundene Pumpeneinheit (12) aufweist, wobei
eine gemeinsame Steuereinheit (66) vorgesehen ist, mit der beide Pumpen (10a,10b) in gegenseitiger Abhängigkeit derart betrieben werden können, dass ihre Volumenströme miteinander gekoppelt sind, dass für jede Pumpe (10a,10b) eine Volumenstrom-Messeinrichtung zur Ermittlung des Volumenstromes (V) dieser Pumpe vorgesehen ist, die mit der Steuereinheit (66) kommuniziert, dass die Volumenstrom-Messeinrichtung mindestens eine Differenzdruck-Messeinrichtung aufweist, die die Druckdifferenz (ΔP1,ΔP2) zwischen der Saugseite der Pumpe und der Druckseite ermittelt, und dass eine Einrichtung vorgesehen ist, die aus der Druckdifferenz (ΔP1,ΔP2) und der Drehzahl der Pumpe den Volumenstrom (V) bestimmt.

2. Pumpvorrichtung nach Anspruch 1, **dadurch gekennzeichnet, dass** eine Pumpe (10a) eine für den rechten Ventrikel (43) bestimmte Rechtsherz-Pumpe und die andere Pumpe (10b) eine für den linken Ventrikel (42) bestimmte Linksherz-Pumpe ist, und dass die Volumenströme beider Pumpen von der gemeinsamen Steuereinheit derart gesteuert sind, dass der Volumenstrom der Linksherz-Pumpe (10b) größer ist als derjenige der Rechtsherz-Pumpe (10a).

3. Pumpvorrichtung nach Anspruch 2, **dadurch gekennzeichnet, dass** der Volumenstrom der Linksherz-Pumpe (10b) um einen bestimmten Prozentsatz, vorzugsweise etwa 10 %, größer ist als derjenige der Rechtsherz-Pumpe (10a).

4. Pumpvorrichtung nach einem der Ansprüche 1-3, **dadurch gekennzeichnet, dass** die Pumpen (10a,10b) jeweils einen mit Wechselstrom von steuerbarer Frequenz betriebenen Elektromotor enthalten.

5. Pumpvorrichtung nach einem der Ansprüche 1-4, **dadurch gekennzeichnet, dass** an jeder Pumpe (10a,10b) mindestens ein Absolutdrucksensor (60,61;62,63) vorgesehen ist, der im Falle eines zu großen oder zu niedrigen Druckes eine Verminderung bzw. Erhöhung des Volumenstromes veranlasst.

6. Pumpvorrichtung nach einem der Ansprüche 1-5, **dadurch gekennzeichnet, dass** die Pumpen (10a,10b) in Master-Slave-Steuerung betrieben sind, wobei die Linksherz-Pumpe (10a) die Funktion des Masters hat und die Rechtsherz-Pumpe (10b) nachgeführt ist.

7. Pumpvorrichtung nach einem der Ansprüche 1-5, **dadurch gekennzeichnet, dass** eine Einrichtung vorgesehen ist, die von beiden Pumpen (10a,10b) diejenige ermittelt, die bei dem jeweiligen Betrieb die förderschwächere ist, und dieser die Master-Funktion zuweist.

8. Pumpvorrichtung nach einem der Ansprüche 1-7, **dadurch gekennzeichnet, dass** mindestens ein Parameter mindestens einer Pumpe (10a,10b) gemessen und auf einen Sollwert geregelt wird.

9. Pumpvorrichtung nach Anspruch 8, **dadurch gekennzeichnet, dass** der Parameter die Druckdifferenz zwischen der Saugseite und der Druckseite der Pumpe ist.

## Claims

1. An intracardiac pump device with two pumps (10a, 10b) to be placed in the heart, each comprising a drive portion (11) and a pump portion (12) rigidly connected therewith, wherein a common control unit (66) controls both pumps (10, 10b) in mutual dependence such that their volume flows are coupled, and wherein, for each pump (10a, 10b), a volume flow measuring means for determining the volume flow (V) of the respective pump is provided, the means communicating with the control unit (66), and the volume flow measuring means comprises at least one differential pressure measuring means determining the differential pressure (ΔP1, ΔP2) between the intake side of the pump and the delivery side, and that means are provided for determining he volume flow (v) from the differential pressure (ΔP1, ΔP2) and the rotational speed of the pump.

2. The pump device of claim 1, **characterized in that** one pump (10a) is a right heart pump for the right ventricle (43) and the other pump (10b) is a left heart pump for the left ventricle (42), and that the volume flows of both pumps are controlled by the common control unit such that the volume flow of the left heart pump (10b) is larger than that of the right heart pump (10b).

3. The pump device of claim 2, **characterized in that** the volume flow of the left heart pump (10b) is larger by a predetermined percentage, preferably about 10%, than that of the right heart pump (10a).

4. The pump device of one of claims 1 - 3, **characterized in that** the pumps (10a, 10b) each comprise an electric motor driven with alternating current of controllable frequency.

5. The pump device of one of claims 1-4, **characterized in that** at least one absolute pressure sensor (60, 61, 62, 63) is provided at each pump (10a, 10b), the sensor causing a reduction or an increase of the volume flow when the pressure is too high or too low.

6. The pump device of one of claims 1-5, **characterized in that** the pumps (10a, 10b) are driven under master-slave control, the left heart pump (10a) taking the function of the master and the right heart pump (10b) following up.

7. The blood pump of one of claims 1-5, **characterized in that** means are provided for determining which of both pumps (10a, 10b) shows a weaker conveying performance under respective operating conditions and for assigning the master function thereto.

8. The pump device of one of claims 1 - 8, **characterized in that** at least one parameter of at least one pump (10a, 10b) is measured and controlled to correspond to a set value.

9. The pump device of claim 8, **characterized in that** the parameter is the differential pressure between the intake side and the delivery side of the pump.

## Revendications

1. Dispositif à pompe intracardiaque comprenant deux pompes (10a, 10b) à placer dans le coeur, chacune présentant une unité d'entraînement (11) et une unité de pompe (12) reliée solidement à cette dernière, dans lequel
on prévoit une unité de commande (66) commune, avec laquelle les deux pompes (10a, 10b) peuvent être mises en fonctionnement en dépendance mutuelle de sorte que leurs débits volumiques sont accouplés ensemble, dans lequel, pour chaque pompe (10a, 10b), on prévoit un appareil de mesure du débit volumique pour déterminer le débit volumique (V) de cette pompe qui communique avec l'unité de commande (66), dans lequel l'appareil de mesure du débit volumique présente au moins un appareil de mesure de pression différentielle qui détermine la différence de pression (ΔP1, ΔP2) entre le côté aspiration de la pompe et le côté refoulement et dans lequel on prévoit un ensemble qui définit le débit volumique (V) à partir de la différence de pression (ΔP1, ΔP2) et de la vitesse de rotation de la pompe.

2. Dispositif à pompe selon la revendication 1,
**caractérisé en ce qu'**une pompe (10a) est une pompe de coeur droit définie pour le ventricule droit (43) et l'autre pompe (10b) est une pompe de coeur gauche définie pour le ventricule gauche (42), et **en ce que** les débits volumiques des deux pompes sont régulés par l'unité de commande commune de telle sorte que le débit volumique de la pompe de coeur gauche (10b) est supérieur à celui de la pompe de coeur droit (10a).

3. Dispositif à pompe selon la revendication 2,
**caractérisé en ce que** le débit volumique de la pompe de coeur gauche (10b) est supérieur d'un pourcentage défini, de préférence environ 10 % à celui de la pompe de coeur droit (10a).

4. Dispositif à pompe selon l'une des revendications 1 à 3,
**caractérisé en ce que** les pompes (10a, 10b) contiennent chacune un moteur électrique entraîné par un courant alternatif à fréquence réglable.

5. Dispositif à pompe selon l'une des revendications 1 à 4,
**caractérisé en ce que**, sur chaque pompe (10a, 10b), on prévoit au moins un capteur de pression absolue (60, 61 ; 62, 63) qui provoque une diminution ou une augmentation du débit volumique en cas d'une pression trop élevée ou trop faible.

6. Dispositif à pompe selon l'une des revendications 1 à 5,
**caractérisé en ce que** les pompes (10a, 10b) sont mises en fonctionnement selon une commande maître-esclave, la pompe de coeur gauche (10a) ayant la fonction de maître et la pompe de coeur droit (10b) étant asservie à celle-ci.

7. Dispositif à pompe selon l'une des revendications 1 à 5,
**caractérisé en ce qu'**on prévoit un ensemble, qui détermine celle des deux pompes (10a, 10b) qui est la plus lente en transport lors de chaque fonctionnement et attribue à celle-ci la fonction de maître.

8. Dispositif à pompe selon l'une des revendications 1 à 7,
**caractérisé en ce qu'**au moins un paramètre d'au moins une pompe (10a, 10b) est mesuré et est asservi sur une valeur de consigne.

9. Dispositif à pompe selon la revendication 8,
**caractérisé en ce que** le paramètre est la différence de pression entre le côté aspiration et le côté refoulement de la pompe.
